(19) Europäisches Patentamt
European Patent Office
Office européen des brevets

(11) Veröffentlichungsnummer: **0 237 918**
**A2**

(12) # EUROPÄISCHE PATENTANMELDUNG

(21) Anmeldenummer: 87103392.4

(22) Anmeldetag: 10.03.87

(51) Int.Cl.³: C 07 C 101/16
A 61 K 31/195

(30) Priorität: 15.03.86 DE 3608725

(43) Veröffentlichungstag der Anmeldung:
23.09.87 Patentblatt 87/39

(84) Benannte Vertragsstaaten:
AT BE CH DE ES FR GB GR IT LI LU NL SE

(71) Anmelder: HOECHST AKTIENGESELLSCHAFT
Postfach 80 03 20
D-6230 Frankfurt am Main 80(DE)

(71) Anmelder: Max-Planck-Gesellschaft zur Förderung der Wissenschaften e.V.
Bunsenstrasse 10
D-3400 Göttingen(DE)

(72) Erfinder: Englert, Heinrich Christian, Dr.
Stormstrasse 13
D-6238 Hofheim am Taunus(DE)

(72) Erfinder: Hropot, Max, Dr.
Friedrich-Stolz-Strasse 13
D-6093 Flörsheim am Main(DE)

(72) Erfinder: Lang, Hans-Jochen, Dr.
Rüdesheimer Strasse 7
D-6238 Hofheim am Taunus(DE)

(72) Erfinder: Greger, Rainer, Prof. Dr.
Im Bremmengässle 3
D-7843 Heitersheim(DE)

(54) **N-Substituierte Aminoalkansäuren, Verfahren zu ihrer Herstellung, ihre Verwendung sowie pharmazeutische Präparate auf Basis dieser Verbindungen.**

(57) Beschrieben wird ein Verfahren zum Herstellen einer Verbindung der Formel I

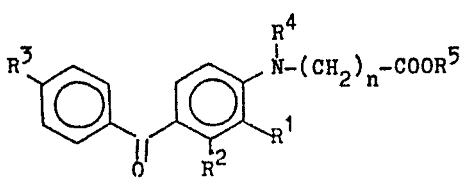

I

in welcher stehen:
$R^1$, $R^2$, $R^3$ (gleich oder verschieden) für H, Alkyl mit 1-2 C-Atomen sowie für F, Cl, Br, J;
$R^4$ für H, Alkyl mit 1-4 C-Atomen,
n für die Zahlen 2-5 und
$R^5$ für einen unter physiologischen Bedingungen abspaltbaren Rest oder für Wasserstoff steht sowie deren physiologisch verträgliche Salze.

Sie sind wirksame Heilmittel gegen Diarrhoe.

EP 0 237 918 A2

HOECHST AKTIENGESELLSCHAFT HOE 86/F 056 Dr.v.F/=
MAX-PLANCK-GESELLSCHAFT ZUR FÖRDERUNG DER WISSENSCHAFTEN E.V.

N-Substituierte Aminoalkansäuren, Verfahren zu ihrer Herstellung, ihre Verwendung sowie pharmazeutische Präparate auf Basis dieser Verbindungen

Die Erfindung betrifft N-substituierte Aminoalkansäuren der Formel I

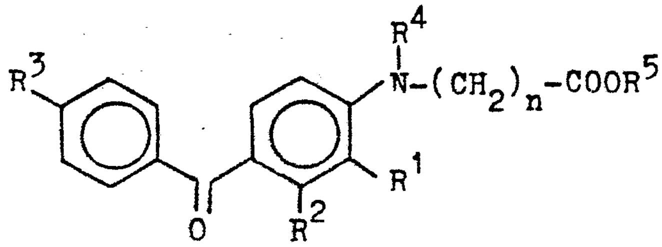

I

in welcher

$R^1$, $R^2$, $R^3$ gleich sind oder verschieden und für H, Alkyl mit 1 - 2 C-Atomen sowie für F, Cl, Br, J, $R^4$ für H, Alkyl mit 1 - 4 C-Atomen, n für die Zahlen 2 - 5 und $R^5$ für Wasserstoff oder einen unter physiologischen Bedingungen abspaltbaren Rest steht sowie deren physiologisch verträgliche Salze.

Verbindungen der Formel I sind neu. Verbindungen, bei denen n für 3 steht, sind formal als N-substituierte γ-Aminobuttersäuren (GABA) aufzufassen. In der Literatur sind zahlreiche Derivate der GABA beschrieben worden, darunter auch solche, die am Aminstickstoff den n-Butyl bzw. 2-Phenylethylrest tragen (Medicinal Research Reviews, Vol. 3, No. 2, 91 - 118 (1983)) und dadurch der erfindungsgemäßen Verbindung I nahekommen bzw. ähnlich sind. Derartige Verbindungen stehen im Zusammenhang mit Struktur-Wirkungsbeziehungen der GABA als Neurotransmitter, wo sie z.T. als schwache GABA-Antagonisten aufgefallen sind. Pharmakologisch ist dies als Hinweis auf eine konvulsive Wirksamkeit der Verbindung aufzufassen.

Es war daher völlig überraschend, daß die erfindungsgemäßen Verbindungen I trotz ihrer Ähnlichkeit mit den oben genannten GABA-Antagonisten anstelle einer unerwünschten krampferzeugenden Wirkung sich vielmehr als geeignet erwiesen zur Behandlung der Diarrhoe, insbesondere solcher Diarrhoeformen, die durch Bakterientoxine wie etwa das Choleratoxin ausgelöst werden. Darüber hinaus werden weitere therapeutische verwertbare Wirkungen wie etwa Salurese, Diurese, ein antipyretischer Effekt als auch eine hemmende Wirkung auf übermäßige Schweißproduktion beobachtet.

Bevorzugt sind Verbindungen der Formel I, bei denen mindestens einer der Substituenten folgende Bedeutung hat: $R^1$, $R^2$, $R^3$ (gleich oder verschieden) Wasserstoff, F, Cl, Br, J, $R^4$ H oder Methyl, $R^5$ $(C_1-C_8)$Alkyl, geradkettig oder verzweigt, sowie n gleich 3.

Die Erfindung betrifft weiterhin ein Verfahren zur Herstellung von N-Arylaminoalkansäuren, dadurch gekennzeichnet, daß man

a) Verbindungen der Formel II

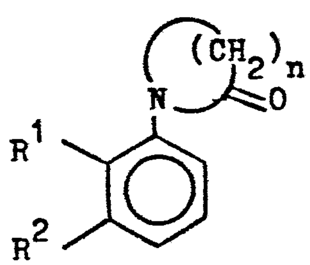

II

benzoyliert zu Verbindungen III

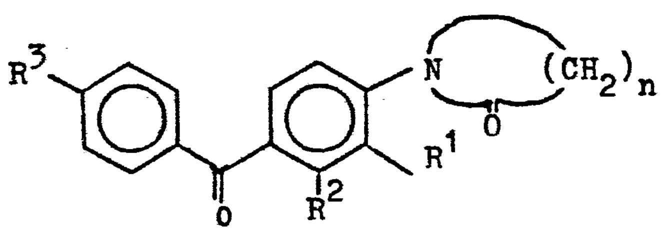

III

und diese hydrolytisch ringöffnet zu Verbindungen I,

b) Verbindungen der Formel IV

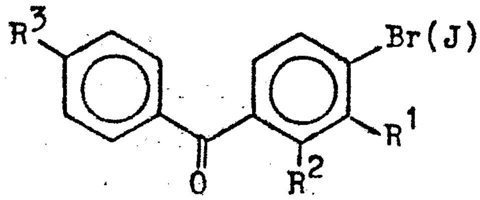

IV

umsetzt mit Lactamen 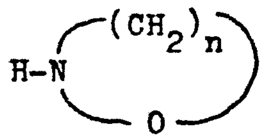

zu Verbindungen III und diese hydrolytisch ringöffnet zu den Verbindungen I

c) Amine V

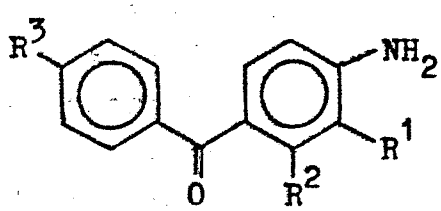

V

umsetzt mit Acrylsäure zu den Verbindungen I

d) Verbindungen der Formel Ia

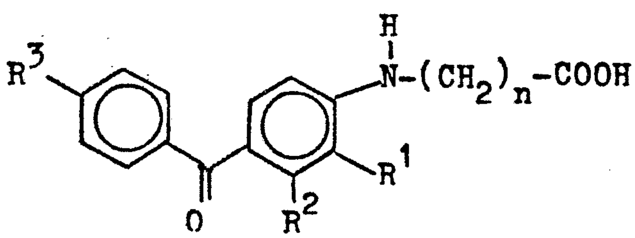

Ia

alkyliert zu Verbindungen Ib

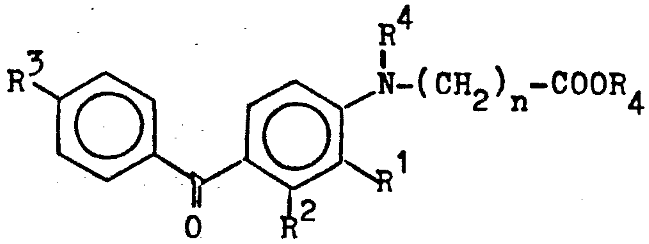

Ib

wobei $R^4$ hier jedoch nicht Wasserstoff bedeutet und diese hydrolysiert zu Verbindungen I.

Bei Verfahren a) wird vorteilhaft so vorgegangen, daß man Verbindungen der Formel II unter Friedel-Crafts-Bedingungen mit Carbonsäurechloriden VIa umsetzt, etwa in Dichlormethan als Lösemittel und mit $AlCl_3$ als Katalysator. Vorteilhaft ist jedoch auch eine Variante, die darin besteht, daß man Carbonsäuren VIb

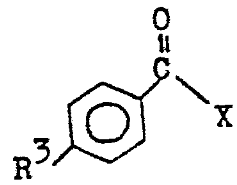

X = Halogen : VIa
X = OH : VIb

in Gegenwart von Polyphosphorsäure unmittelbar mit den Verbindungen II umsetzt. Die Ringöffnung von II geschieht am vorteilhaftesten dadurch, daß man II in einem geeigneten organischen Lösemittel wie etwa Methanol oder Ethanol löst und dann soviel wässerige Alkalihydroxidlösung zusetzt bis II gerade auszufallen beginnt. Die Mischung wird dann bis zur vollständigen Ringöffnung erwärmt, vorteilhaft bei der Rückflußtemperatur des verwendeten Lösemittels.

Verbindungen III können auch dadurch hergestellt werden, daß man 4-Brom-oder 4-Jodbenzophenonderivate IV nach Art der Ullmann-Goldberg-Reaktion umsetzt mit Lactamen

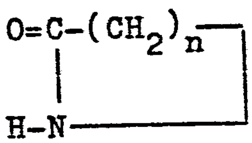

unter Cu-Katalyse.

Man verwendet inerte Lösemittel wie Dimethylformamid oder verzichtet vorteilhaft ganz auf ein Lösemittel oder verwendet überschüssiges Lactam als Lösemittel, was sich in der Praxis als ganz besonders vorteilhaft erwiesen hat. Auch der Zusatz einer Base, beispielsweise von fein gepulvertem Kaliumacetat oder Kaliumcarbonat ist für gute Ausbeuten vorteilhaft. Für die Umsetzung sind im allgemeinen höhere Temperaturen notwendig, vorteilhaft wird im Bereich von 140 – 190°C gearbeitet.

Bei der Verfahrensvariante c) wird das Amin V am besten in Gegenwart schwacher Alkansäuren wie Essigsäure oder Propionsäure mit der Acrylsäure umgesetzt. Die Reaktionen erfordern im allgemeinen erhöhte Reaktionstemperaturen, z.B. Rückflußtemperatur der zugesetzten Alkansäure.

Bei Verfahrensvariante d) werden Verbindungen I, bei denen $R^4$ für H steht, umgesetzt mit 2 Äquivalenten eines Alkylierungsmittels $R^4$-Y - wobei Y für eine Fluchtgruppe steht wie Cl, Br, J, Tosylat, Mesylat, Methylsulfat. Diese Reaktion wird nach Standardmethoden ausgeführt, im allgemeinen wird zunächst die Carboxylgruppe in den Ester überführt, und erst nach längerer Reaktionszeit erhält man auch die gewünschte N-Alkylierung. Man ist dabei insbesondere auf ganz schwache Basen als Katalysator bei dieser Reaktion angewiesen . So führt $NaHCO_3$ in Dimethylformamid zu den gewünschten Produkten, während z.B. $K_2CO_3$ in DMF zu den hier unerwünschten Verbindungen III führen würde. Die Verseifung der Verbindungen Ib erfolgt alkalisch nach Standardmethoden, vorteilhaft mit wässeriger Lithiumhydroxydlösung.

Die erfindungsgemäßen Verbindungen der Formel I sowie deren pharmazeutisch verträgliche Salze - es kommen hier vor allem die Alkali- und Erdalkalisalze in Betracht, wie beispielsweise $Na^+$, $K^+$, $NH_4^+$ oder $Ca^{++}$-Salze, aber auch Salze organischer Basen, wie beispielsweise das Ethanolaminsalz sind von Bedeutung - sind Mittel zur Behandlung der Diarrhoe. Sie werden in Dosierungen von mindestens 0,01 mg/kg, vorzugsweise 0,05 mg/kg und insbesondere 0,5 mg/kg bis maximal 200 mg/kg, vorzugsweise 50 mg/kg und insbesondere 20 mg/kg Körpergewicht, bezogen auf einen Erwachsenen von 75 kg Gewicht, in Kapseln, Dragees, Tabletten oder Lösung allein oder in Kombination mit Elektrolytlösungen, die der Dehydratation bei Diarrhoe entgegenwirken, enteral z.B. oral, verabfolgt. Sie eignen sich zur Behandlung aller Krankheiten, bei denen es zu

einem pathologisch gesteigerten Verlust von Wasser und Elektrolyten über den Darm kommt wie dies bei den verschiedensten Formen der Diarrhoe auftritt, vor allem bei der toxisch bedingten Diarrhoe infolge von Infektionskrankheiten wie etwa Cholera oder erblich bedingten Diarrhoeformen wie der kongenitalen Chloriddiarrhoe.

Beispiel 1:

4-(4-Benzoylanilino)buttersäure

2,61 g (0,01 Mol) 4-Brombenzophenon und 1,13 ml Butyrolactam (0,015 Mol) werden bei 180°C zusammengeschmolzen, mit 1,4 g $K_2CO_3$ sowie mit 0,63 g Cu-Pulver versetzt. Man rührt 2 h bei dieser Temperatur, läßt auf 140°C abkühlen und bringt die Mischung mit Essigsäureethylester in Lösung. Chromatographie an Kieselgel mit dem Elutionsmittel Toluol/Essigsäureethylester liefert das N-(4-Benzoylphenyl)-pyrrolidin-2-on vom Schmp.: 162 - 164°C, das sofort in 10 ml Methanol sowie 10 ml 5 N NaOH gelöst wird und ca. 2 h am Rückfluß gekocht wird. Nach Abdestillieren des Methanols wird auf pH 5 angesäuert und das Produkt abgesaugt. Schmp.: 156-158°C

Beispiel 2:

5-(4-Benzoylanilino)valeriansäure

Analog Beispiel 1 mit Valerolactam anstelle von Butyrolactam.
Schmp.: 123 - 124°C

Beispiel 3:

3-(4-Benzoylanilino)propionsäure

1,97 g 4-Aminobenzophenon (0,01 Mol) und 1,44 g Acrylsäure (0,02 Mol) werden in 10 ml Eisessig gelöst und 2 Tage am

Rückfluß gekocht. Die Lösung wird im Vakuum bis zur Trockene eingeengt, mit 2 N Natronlauge erneut in Lösung gebracht und dann mehrfach mit Diethylether extrahiert. Die wässerige Lösung wird mit 2 N HCl auf pH 4 - 5 gebracht und der Niederschlag abgesaugt und mit Wasser gewaschen.
Schmp.: 139 - 141°C

Beispiel 4:

4-[4-(4-Methoxybenzoyl)anilino]buttersäure

1,61 g (0,01 Mol) N-Phenylpyrrolidin-2-on und 1,49 g (0,011 Mol) 4-Methylbenzoesäure wurden in 20 ml Polyphosphorsäure bei 170°C 1 h gerührt. Man gießt die Mischung auf Eiswasser, saugt ab, löst den Niederschlag in Ethylacetat. Die organische Phase wird 3 mal mit gesättigter $NaHCO_3$-Lösung extrahiert, mit $MgSO_4$ getrocknet, über Kieselgel filtriert (Elution mit Ethylacetat) und im Vakuum zur Trockne eingeengt. Der Rückstand wird aus Toluol umkristallisiert und anschließend wie unter Beispiel 1 zur Titelverbindung verseift.
Schmp. 185 - 186°C

Beispiel 5:

4-[4-(4-Chlorbenzoyl)anilino]buttersäure

Analog Beispiel 4 erhält man aus 4-Chlorbenzoesäure und N-Phenylpyrrolidin-2-on die Titelverbindung mit Schmp.: 172 - 173°C

Beispiel 6:

4-(4-Benzoyl-3-chloranilino)buttersäure

Analog Beispiel 4 erhält man aus Benzoesäure und N-(3-Chlorphenyl)pyrrolidin-2-on die Titelverbindung mit Schmp.: 136 - 137°C

Beispiel 7:

4-(4-Benzoyl-2-chloranilino)buttersäure

Analog Beispiel 4 erhält man aus Benzoesäure und N-(2-Chlorphenyl)pyrrolidin-2-on die Titelverbindung mit Schmp.: 160 - 161°C

Beispiel 8:

4-(N-Methyl-4-Benzoylanilino)buttersäure

1,41 g (0,05 Mol) 4-(4-Benzoylanilino)buttersäure werden in 15 ml DMF gelöst, mit 0,84 g $NaHCO_3$ (0,01 Mol) sowie mit 1,42 g (0,01 Mol) Methyljodid versetzt und ca. 3 h bei 80°C gerührt. Man gießt auf Eiswasser, extrahiert mit Ethylacetat, trocknet die organische Phase mit $MgSO_4$ und verdampft das Lösemittel im Vakuum. Der Rückstand wird an Kieselgel mit dem Elutionsmittel n-Hexan/Ethylacetat chromatographiert. Das ölige Produkt wird sofort der Verseifung mit 2 N LiOH unterworfen. Man erhält die Titelverbindung vom Schmp.: 111 - 113°C

Ansprüche:

1. Verbindung der Formel I

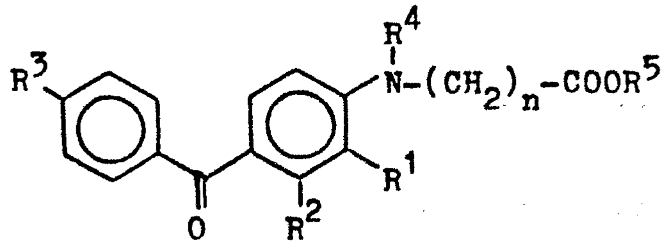

I

in welcher

$R^1$, $R^2$, $R^3$ gleich sind oder verschieden und für H, Alkyl mit 1 - 2 C-Atomen sowie für F, Cl, Br, J, $R^4$ für H, Alkyl mit 1 - 4 C-Atomen, n für die Zahlen 2 - 5 und $R^5$ für Wasserstoff oder einen unter physiologischen Bedingungen abspaltbaren Rest steht sowie deren physiologisch verträgliche Salze.

2. Verbindung I nach Anspruch 1, bei der mindestens einer der Substituenten folgende Bedeutung hat: $R^1$, $R^2$, $R^3$ (gleich oder verschieden) Wasserstoff, F, Cl, Br, J, $R^4$ H oder Methyl, $R^5$ Wasserstoff oder ($C_1$-$C_8$) Alkyl geradkettig oder verzweigt sowie n gleich 3.

3. Verfahren zum Herstellen von Verbindungen I nach Anspruch 1, dadurch gekennzeichnet, daß man

a) Verbindungen der Formel II

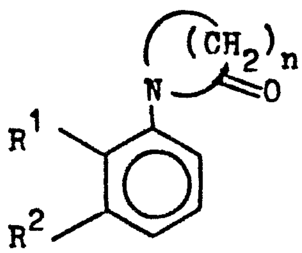

II

benzoyliert zu Verbindungen III

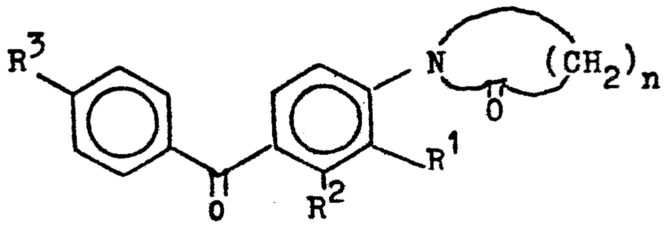

III

und diese hydrolytisch ringöffnet zu Verbindungen I,

b) Verbindungen der Formel IV

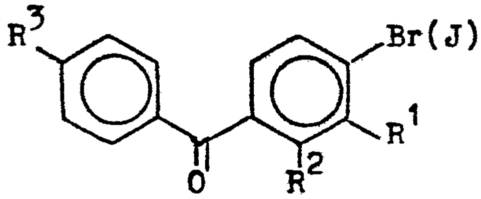

IV

umsetzt mit Lactamen 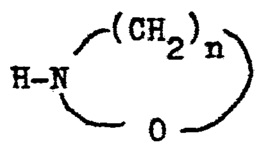

zu Verbindungen III und diese hydrolytisch ringöffnet zu den Verbindungen I

c) Amine V

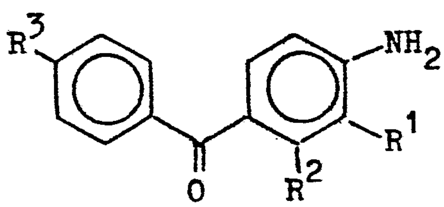

V

umsetzt mit Acrylsäure zu den Verbindungen I

d) Verbindungen der Formel Ia

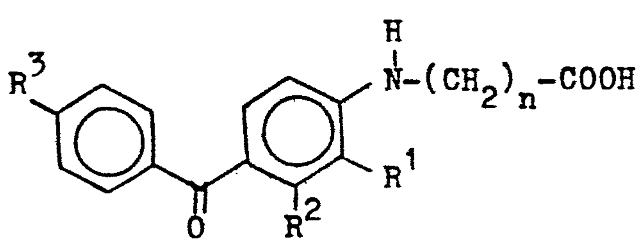

Ia

alkyliert zu Verbindungen Ib

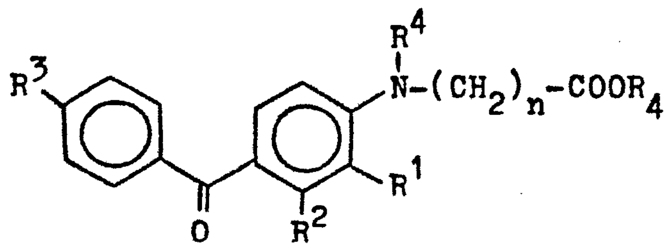

Ib

wobei $R^4$ hier jedoch nicht Wasserstoff bedeutet, und diese hydrolysiert zu Verbindungen I.

4. Verwendung einer Verbindung der Formel I nach Anspruch 1 als Heilmittel gegen Diarrhoe.

5. Verwendung einer Verbindung der Formel I nach Anspruch 1 zur Herstellung eines antidiarrhoeisch wirkenden Arzneimittels.

6. Arzneimittel mit antidiarrhoeischer Wirkung, gekennzeichnet durch einen wirksamen Gehalt einer Verbindung I nach Anspruch 1.

Patentansprüche Griechenland, Spanien und Österreich:

1. Verfahren zum Herstellen einer Verbindung I,

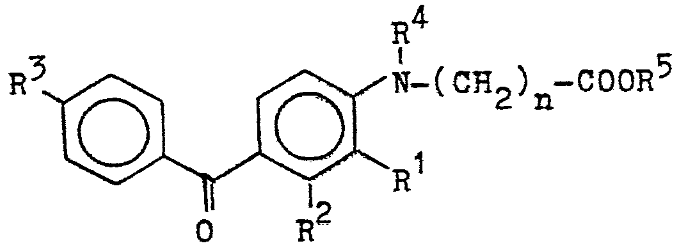

I

in welcher
$R^1$, $R^2$, $R^3$ gleich sind oder verschieden und für H, Alkyl, mit 1-2 C-Atomen sowie für F, Cl, Br, J,
$R^4$ für H, Alkyl mit 1-4 C-Atomen,
n für die Zahlen 2-5 und
$R^5$ für Wasserstoff oder einen unter physiologischen Bedingungen abspaltbaren Rest steht
sowie von deren physiologisch verträglichen Salzen, dadurch gekennzeichnet, daß man

a) eine Verbindung der Formel II

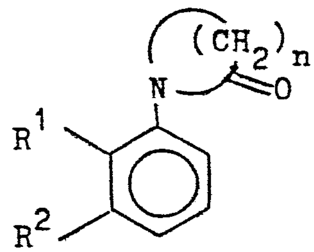

II

benzoyliert zu einer Verbindung III

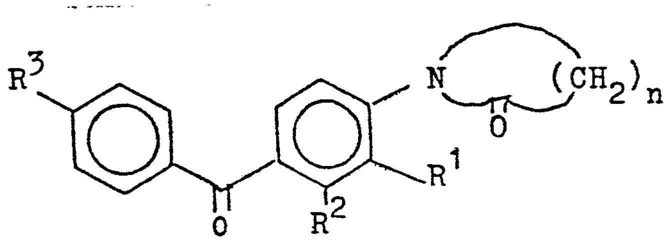

III

und diese hydrolytisch ringöffnet zu einer Verbindung I, und gegebenenfalls in Salze mit physiologisch verträglichen Säuren überführt;

b) eine Verbindung der Formel IV

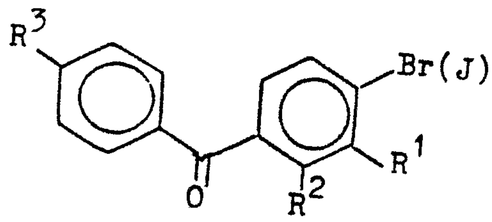

IV,

in der $R^1$, $R^2$ und $R^3$ wie oben definiert sind,

umsetzt mit einem Lactamen 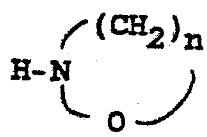

zu einem Verbindung III und diese hydrolytisch ringöffnet zur Verbindung I, und gegebenenfalls in Salze mit physiologisch verträglichen Säuren überführt;

c) ein Amin V 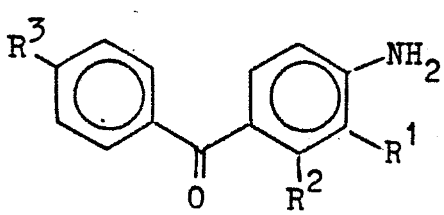 V

in dem $R^1$, $R^2$ und $R^3$ wie oben definiert sind, umsetzt mit Acrylsäure zu einer Verbindung I, und gegebenenfalls in Salze mit physiologisch verträglichen Säuren überführt;

d) einer Verbindung der Formel Ia

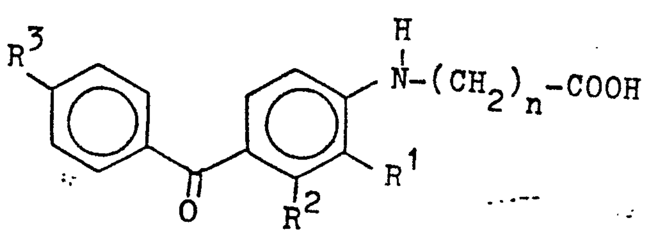

Ia

in der $R^1$, $R^2$ und $R^3$ wie oben definiert sind, alkyliert zu einer Verbindung Ib

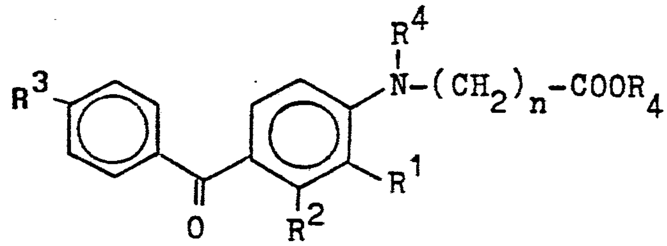

Ib,

wobei $R^4$ hier jedoch nicht Wasserstoff bedeutet, und diese hydrolysiert zur Verbindung I, und gegebenenfalls in Salze mit physiologisch verträglichen Säuren überführt;

2. Verfahren nach Anspruch 1, dadurch gekennzeichnet, daß mindestens einer der Substituenten folgende Bedeutung hat: $R^1$, $R^2$, $R^3$ (gleich oder verschieden) Wasserstoff, F, Cl, Br, J; $R^4$, H oder Methyl; $R^5$ Wasserstoff oder $(C_1-C_8)$Alkyl geradkettig oder verzweigt sowie n gleich 3.

3. Verwendung einer nach Anspruch 1 erhaltenen Verbindung der Formel I als Heilmittel gegen Diarrhoe.

4. Verwendung einer nach Anspruch 1 erhaltenen Verbindung der Formel I zur Herstellung eines antidiarrhoeisch wirkenden Arzneimittels.